# FASCICULE DE BREVET EUROPEEN

(11) **EP 1 700 087 B1**
(45) Date de publication et mention de la délivrance du brevet: **15.02.2012**
(21) Numéro de dépôt: 04816559.1
(22) Date de dépôt: 16.12.2004
(51) Int. Cl.: G01F 13/00, A61M 15/00

(54) **DISPOSITIF DE DISTRIBUTION DE PRODUIT FLUIDE**
FLUIDPRODUKTABGABEVORRICHTUNG
FLUID PRODUCT DISPENSING DEVICE

(30) Priorité: 19.12.2003 FR 0315040
(43) Date de publication de la demande: 13.09.2006
(60) Demande divisionnaire: 11170173.6
(73) Titulaire: Valois SAS, 27110 Le Neubourg (FR)
(72) Inventeur: BRUNA, Pascal, F-76300 Sotteville Les Rouen (FR); SAUZADE, Jean-Denis, F-06130 Grace (FR)
(74) Mandataire: CAPRI
(86) Numéro de dépôt international: PCT/FR2004/050706
(87) Numéro de publication internationale: WO 2005/061998

(56) Documents cités:
- WO-A-02/070047
- GB-A- 2 116 314
- GB-A- 2 304 327
- US-A- 5 415 161
- US-A- 5 433 342
- US-A- 6 119 684
- US-A- 6 129 702
- US-A- 6 138 669

## Description

La présente invention concerne un dispositif de distribution de produit fluide, et plus particulièrement un tel dispositif comportant une pompe ou une valve actionnée manuellement.

Il est connu d'utiliser des pompes ou des valves pour distribuer de manière dosée du produit fluide (liquide, crème ou poudre), notamment dans le domaine de la pharmacie, de la parfumerie et de la cosmétique. En particulier dans le domaine de la pharmacie, il peut être très important d'éviter tous risques de surdosage et/ou de sous-comptage. Avec une valve, fonctionnant au moyen d'un gaz propulseur, le problème concerne surtout le comptage des doses émises, et il est souvent nécessaire d'éviter tout risque de sous-comptage pour éviter que l'utilisateur ne se retrouve avec un dispositif vide alors qu'il pense qu'il reste quelques doses à distribuer. En effet, des systèmes de comptage sont généralement associés à ces valves pour compter les doses émises, ces systèmes étant généralement actionnés par le déplacement relatif entre le réservoir et la soupape de la valve. Des problèmes peuvent se poser en cas d'actionnement partiel ou interrompu, qui peut provoquer une expulsion de dose, partielle ou non. Des compteurs compliqués ont été proposés pour prendre en compte ce problème, mais leur fiabilité impose une complexité et donc un coût élevé. Lorsque l'on utilise une pompe, et notamment les pompes de nouvelle génération, il peut se poser un autre problème en plus du risque de sous-comptage. En effet, la pulvérisation peut être tellement fine que la distribution de la dose n'est pas toujours ressentie par l'utilisateur. Ceci est notamment vrai avec certaines pompes de distribution nasales. Dans ce cas, si il n'y a aucune indication donnée à l'utilisateur pour lui indiquer que la dose à bien été distribuée, il y'a un risque que l'utilisateur actionne à nouveau le dispositif en pensant que son premier actionnement a été inefficace. Ceci entraîne un risque de surdosage, qui peut être néfaste pour sa santé.

La présente invention a pour but de fournir un dispositif de pulvérisation de produit fluide qui ne reproduit pas les inconvénients susmentionnés.

En particulier, la présente invention a pour but de fournir un dispositif de pulvérisation de produit fluide comportant une pompe ou une valve qui évite tout risque de surdosage et/ou de sous-comptage des doses émises.

La présente invention a également pour but de fournir un tel dispositif qui soit simple et peu coûteux à fabriquer et à assembler, ainsi que sûre et fiable à utiliser.

La présente invention a donc pour objet un dispositif de distribution de produit fluide tel que décrit dans la revendication 1. Des modes de réalisation avantageux sont décrits dans les revendications dépendantes.

D'autres caractéristiques et avantages de la présente invention apparaîtront plus clairement au cours de la description détaillée suivante faite en référence aux dessins joints, donnés à titre d'exemples non limitatifs, et sur lesquels :
- la figure 1 est une vue schématique en section transversale d'un dispositif de pulvérisation selon un mode de réalisation de la présente invention,
- la figure 2 est une vue de détail agrandie d'une partie de la figure 1,
- la figure 3 est une vue similaire à celle de la figure 1, montrant une variante de réalisation de la présente invention,
- la figure 4 est une vue similaire à celle de la figure 1, montrant un autre mode de réalisation, et
- la figure 5 est une vue de détail agrandie d'une partie de la figure 4.

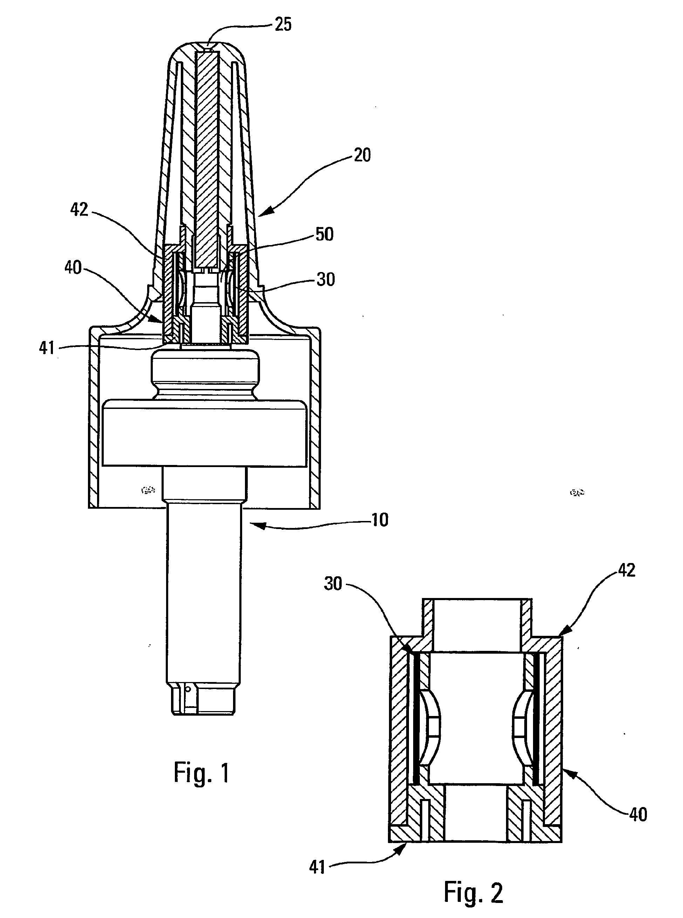

En référence à la figure 1, il est représenté un organe de distribution qui est une pompe 10, sur laquelle est assemblée une tête de distribution 20 pourvue d'un orifice de distribution 25. La pompe peut être d'un type quelconque, et la structure interne de cette pompe ne sera donc pas décrite plus amplement ci-après. La présente invention s'applique notamment, mais pas exclusivement, aux pompes de distribution qui sont adaptées à distribuer de manière très finement pulvérisée une dose de produit fluide à chaque actionnement. La tête de distribution 20 représentée sur la figure 1 est une tête de distribution nasale, et elle est utilisée pour actionner manuellement la pompe 10. Bien entendu, la présente invention n'est pas limitée à l'exemple représenté sur la figure, mais différentes variantes de réalisation sont envisageables. La pompe 10 est destinée à être assemblée sur un réservoir (non représenté), d'une manière quelconque connue.

Selon l'invention, le dispositif comporte des moyens de détection de distribution 30, 31 qui sont adaptés à détecter la distribution d'une dose de produit. Ces moyens de détection 30, 31 sont de préférence adaptés à délivrer un signal utilisé pour informer l'utilisateur qu'une dose de produit a été effectivement distribuée par ladite pompe. L'information de l'utilisateur peut être réalisée de diverses manières, par exemple au moyen d'un dispositif d'affichage. En variante, on pourrait envisager d'utiliser des moyens d'informations sonores ou similaires pour indiquer à l'utilisateur la distribution de la dose. Avantageusement, le signal émis par les moyens de détection de distribution 30 pourrait également être utilisé pour actionner un compteur de doses. Ainsi, dans le cas d'une pompe de distribution dans laquelle la dose est si finement pulvérisée que l'utilisateur ne se rend pas compte de cette distribution, la présente invention permet d'éviter tout risque de surdosage en informant l'utilisateur que la dose a bien été distribuée.

Selon un mode de réalisation, ces moyens de détection comportent un détecteur de pression dynamique 30 pour détecter le passage de produit entre la pompe 10 et l'orifice de distribution 25.

Le détecteur 30 comporte de préférence un matériau piézoélectrique, avantageusement du PVDF (PolyVynil DiFluoré), qui est un matériau plastique possédant des propriétés piézoélectriques, Avantageusement, comme visible sur la figure 2, le détecteur 30 comporte un tube de PVDF fonctionnant en mode respiration.

Cette mise en oeuvre garantit une détection parfaitement fiable, même en cas de très faibles quantités expulsées.

Comme représenté sur la figure 1, le détecteur 30 peut être disposé dans le canal d'expulsion 50 qui relie la pompe de distribution 10 à l'orifice de pulvérisation 25 du dispositif. Avantageusement, il est disposé dans un manchon 40 relié d'une part à la tige d'actionnement de la pompe 10 et d'autre part à la tête de distribution 20. Ce manchon 40 peut être emmanché autour de cette tige d'actionnement, et il peut être formé en deux parties 41, 42 emboîtées l'une sur ou autour de l'autre, le détecteur 30 étant disposé entre ces deux parties. Ceci simplifie et facilite l'assemblage du dispositif, et permet de garantir l'étanchéité.

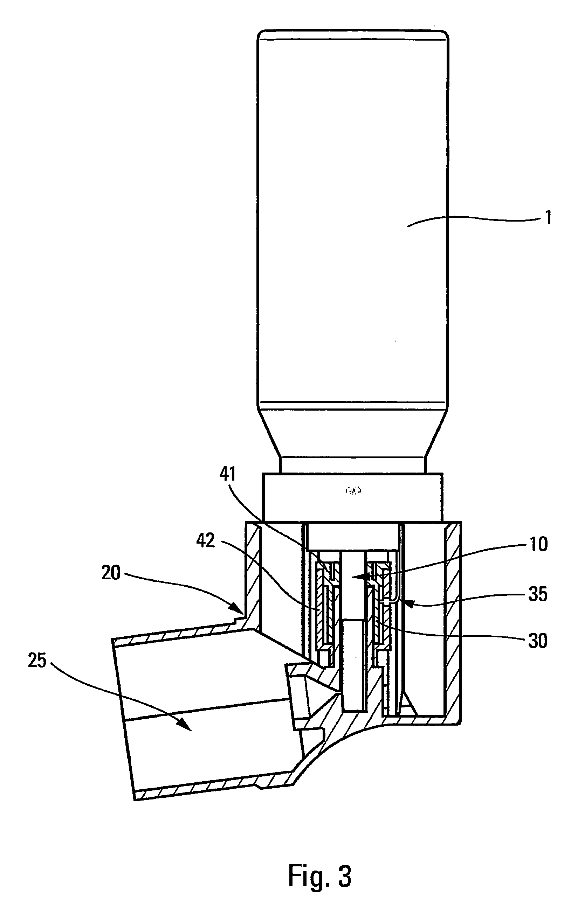

La figure 3 montre une variante de réalisation, dans lequel l'organe de distribution est une valve 10, par exemple une valve doseuse fonctionnant en position inversée. Le détecteur 30 est mis en oeuvre de manière similaire à l'exemple précédent, à savoir sous la forme d'un tube PVDF placé dans un manchon 40 réalisé en deux parties 41, 42, et assemblé autour du puit de soupape de la valve. Dans cette application, le produit fluide est généralement une solution aqueuse, la pression maximale en sortie de la valve pouvant aller jusqu'à 1000 kPa (10 bars). Ceci implique des contraintes élevées sur le détecteur ainsi que pour l'étanchéité, et la structure décrite ci-dessus permet de s'adapter sans problème à ces contraintes pour assurer un fonctionnement fiable du dispositif de distribution, évitant tout sous-comptage et tout surdosage, même en cas d'actionnement partiel du dispositif.

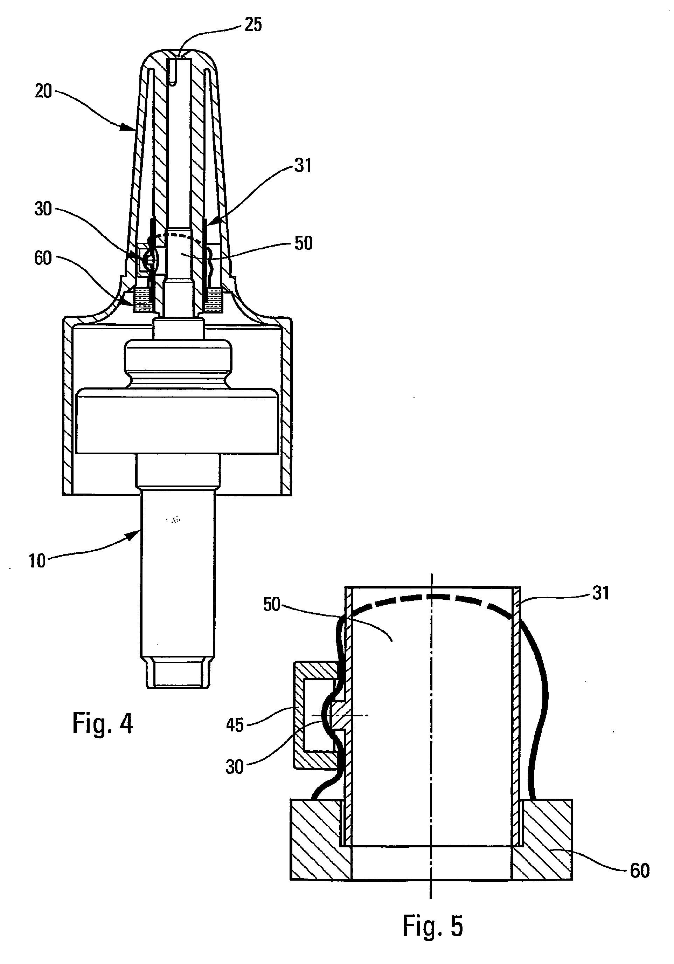

La figure 4 montre un autre mode de réalisation, dans lequel les moyens de détection comportent un détecteur formé par une fibre optique 30. Cette fibre optique est de préférence associée à une membrane déformable 31, avantageusement disposée autour d'une partie du canal d'expulsion 50. Lors de l'expulsion de produit fluide, la membrane 31 se déforme, notamment sous l'effet de la pression, ce qui génère des contraintes dans la fibre optique 30, créant ainsi un signal exploitable par des moyens électroniques 60 appropriés. La mesure optique est avantageuse en terme de miniaturisation dans la mesure ou l'on utilise une fibre optique compatible avec les techniques de moulage et/ou surmoulage plastique. Avantageusement, la fibre optique 30 coopère avec la membrane 31 dans un carter 45 qui peut être solidaire de la tête de distribution 20, et dans lequel la fibre optique 30 est retenue fixement de manière à détecter tout déformation de la membrane 31. Dans cet exemple de réalisation, les moyens électriques 60 peuvent comporter un émetteur et un détecteur sensible à la variation de flux lumineux transmis pas la fibre optique 30, variation due à la contrainte générée dans la fibre. La fibre optique peut être un plastique ou en verre.

De manière générale, les moyens de détection 30, 31 peuvent être connectés, via des moyens de connexion adaptés 35, à des moyens électroniques (60) qui sont adaptés à traiter le ou les signaux délivrés par ledit détecteur 30, pour informer l'utilisateur de la distribution de la dose, et/ou actionner un compteur ou indicateur de doses.

Bien que la présente invention ait été décrite en référence à des modes de réalisation particuliers de celle-ci, il est entendu qu'elle n'est pas limitée par les exemples représentés sur les figures. Au contraire, un homme du métier peut y apporter toutes modifications utiles sans sortir du cadre de la présente invention tel que défini par les revendications annexées.

## Revendications

1. Dispositif de distribution de produit fluide comportant un organe de distribution de produit fluide (10), tel qu'une pompe ou une valve, et une tête de distribution (20) comportant un orifice de distribution, l'organe de distribution (10) étant relié à l'orifice de distribution (40) par un canal d'expulsion (50), ledit dispositif comportant des moyens de détection de distribution (30, 31) pour détecter la distribution d'une dose de produit, lesdits moyens de détection (30, 31) étant prévus dans ledit canal d'expulsion (50), lesdits moyens de détection (30, 31) étant adaptés à délivrer un signal pour informer l'utilisateur qu'une dose de produit à été effectivement distribuée par ledit organe de distribution (10), lesdits moyens de détection comprenant un détecteur (30) pour détecter le passage du produit fluide à partir dudit organe de distribution vers ledit orifice de distribution, **caractérisé en ce que** ledit détecteur (30) comporte un matériau piézoélectrique, et **en ce que** lesdits moyens de détection (30, 31) sont disposés dans un manchon (40) coopérant d'une part avec ledit organe de distribution (10) et d'autre part avec ladite tête de distribution (20), ledit manchon (40) étant réalisé en deux parties (41, 42) emmanchées l'une sur et/ou autour de l'autre, lesdits moyens de détection (30, 31) étant disposés entre lesdites deux parties de manchon (41, 42).

2. Dispositif selon la revendication 1, dans lequel ledit détecteur (30) est un détecteur de pression dynamique.

3. Dispositif selon l'une quelconque des revendications précédentes, dans lequel ledit détecteur (30) comporte du PVDF (PolyVynil DiFluoré).

4. Dispositif selon l'une quelconque des revendications précédentes, dans lequel ledit détecteur (30) comporte un tube de PVDF.

5. Dispositif selon la revendication 4, dans lequel ledit tube PVDF est disposé autour d'une partie dudit canal d'expulsion (50).

6. Dispositif selon l'une quelconque des revendications 1 à 5, dans lequel ledit manchon (40) est emmanché autour de la soupape de la valve, respectivement de la tige d'actionnement de la pompe.

7. Dispositif selon l'une quelconque des revendications précédentes, dans lequel lesdits moyens de détection (30, 31) sont connectés à des moyens électroniques (60) pour traiter les signaux délivrés par lesdits moyens de détection (30, 31).

8. Dispositif selon l'une quelconque des revendications précédentes, dans lequel lesdits moyens de détection (30, 31) sont adaptés à incrémenter, respectivement décrémenter, un compteur de doses.

9. Dispositif selon l'une quelconque des revendications précédentes, dans lequel ledit organe de distribution est une pompe (10) adaptée à distribuer le produit de manière très finement pulvérisée.

10. Dispositif selon l'une quelconque des revendications 1 à 8, dans lequel ledit organe de distribution est une valve doseuse (10) fonctionnant avec un gaz propulseur.

## Claims

1. A fluid dispenser device including a fluid dispenser member (10) such as a pump or a valve, and a dispenser head (20) provided with a dispensing orifice, the dispenser member (10) being connected to the dispensing orifice (40) via an expulsion channel (50), said fluid dispenser device being provided with dispensing detector means (30, 31) for detecting dispensing of a dose of fluid, said detector means (30, 31) being provided in said expulsion channel (50), said detector means (30, 31) being adapted to deliver a signal for informing the user that a dose of fluid has indeed been dispensed by said dispenser member, said detector means comprising a detector (30) for detecting the fluid going from said dispenser member to said dispenser orifice, said fluid dispenser device being **characterized in that** said detector (30) comprises a piezoelectric material, and **in that** said detector means (30, 31) are disposed in a sleeve (40) co-operating with said dispenser member (10) and with said dispenser head (20), said sleeve (40) being made up of two portions (41, 42) engaged one on and/or around the other, said detector means (30, 31) being disposed between said two sleeve portions (41, 42).

2. A device according to claim 1, in which said detector (30) is a dynamic pressure detector.

3. A device according to any preceding claim, in which said detector (30) comprises polyvinylidene fluoride (PVDF).

4. A device according to any preceding claim, in which said detector (30) comprises a PVDF tube.

5. A device according to claim 4, in which said PVDF tube is disposed around a portion of said expulsion channel (50).

6. A device according to any one of claims 1 to 5, in which said sleeve (40) is engaged around the valve member of the valve, or around the actuating rod of the pump.

7. A device according to any preceding claim, in which said detector means (30, 31) are connected to electronic means (60) for processing the signals delivered by said detector means (30, 31).

8. A device according to any one of preceding claim, in which said detector means (30, 31) are adapted to increment or to decrement a dose counter.

9. A device according to any preceding claim, in which said dispenser member is a pump (10) adapted to dispensing the fluid such that it is very finely sprayed.

10. A device according to any one of claims 1 to 8, in which said dispenser member is a metering valve (10) operating with a propellant gas.

## Patentansprüche

1. Abgabevorrichtung für ein fluides Produkt, aufweisend eine Abgabeeinrichtung für ein fluides Produkt (10), wie eine Pumpe oder ein Ventil, und einen Abgabekopf (20), der eine Abgabeöffnung aufweist, wobei die Abgabeeinrichtung (10) durch einen Austreibkanal (50) mit der Abgabeöffnung (40) verbunden ist, wobei die Vorrichtung Mittel zum Erkennen der Abgabe (30, 31) aufweist, um die Abgabe einer Produktdosis zu erkennen, wobei die Erkennungsmittel (30, 31) in dem Austreibkanal (50) vorgesehen sind, wobei die Erkennungsmittel (30, 31) dazu geeignet sind, ein Signal abzugeben, um den Benutzer darüber zu informieren, dass eine Produktdosis von der Abgabeeinrichtung (10) wirksam abgegeben wurde, wobei die Erkennungsmittel einen Sensor (30) zum Erkennen des Fließens des fluiden Produktes von der Abgabeeinrichtung über die Abgabeöffnung aufweisen, **dadurch gekennzeichnet, dass** der Sensor (30) ein piezoelektrisches Material aufweist, und dass die Erkennungsmittel (30, 31) in einer Hülse (40) angeordnet sind, die einerseits mit der Abgabeeinrichtung (10) und andererseits mit dem Abgabekopf (20) zusammenwirkt, wobei die Hülse (40) in zwei Teilen (41, 42) ausgeführt ist, wobei das eine Teil auf und/oder um das andere Teil gesetzt ist, wobei die Erkennungsmittel (30, 31) zwischen den beiden Teilen der Hülse (41, 42) angeordnet sind.

2. Vorrichtung nach Anspruch 1, wobei der Sensor (30) ein dynamischer Drucksensor ist.

3. Vorrichtung nach einem der vorhergehenden Ansprüche, wobei der Sensor (30) PVDF (Polyvinylidenfluorid) aufweist.

4. Vorrichtung nach einem der vorhergehenden Ansprüche, wobei der Sensor (30) eine PVDF-Röhre aufweist.

5. Vorrichtung nach Anspruch 4, wobei die PVDF-Röhre um einen Teil des Austreibkanals (50) angeordnet ist.

6. Vorrichtung nach einem der Ansprüche 1 bis 5, wobei die Hülse (40) um den Ventilregler bzw. die Betätigungsstange der Pumpe gesetzt ist.

7. Vorrichtung nach einem der vorhergehenden Ansprüche, wobei die Erkennungsmittel (30, 31) mit elektronischen Mitteln (60) verbunden sind, um die von den Erkennungsmitteln (30, 31) abgegebenen Signale zu verarbeiten.

8. Vorrichtung nach einem der vorhergehenden Ansprüche, wobei die Erkennungsmittel (30, 31) dazu geeignet sind, einen Dosiszähler zu inkrementieren bzw. dekrementieren.

9. Vorrichtung nach einem der vorhergehenden Ansprüche, wobei die Abgabeeinrichtung eine Pumpe (10) ist, die dazu geeignet ist, das Produkt sehr fein pulverisiert abzugeben.

10. Vorrichtung nach einem der Ansprüche 1 bis 8, wobei die Abgabeeinrichtung ein Dosierventil (10) ist, das mit Treibgas arbeitet.
